# EUROPEAN PATENT SPECIFICATION

(11) **EP 3 053 535 B1**
(45) Date of publication and mention of the grant of the patent: **15.01.2025**
(21) Application number: 16154539.7
(22) Date of filing: 05.02.2016
(51) Int. Cl.: A61B 17/80

(54) **BONE PLATE WITH INSERT(S) FOR OPTIMALLY DIRECTING FASTENERS**
KNOCHENPLATTE MIT EINSATZ(EN) ZUR OPTIMALEN FÜHRUNG VON BEFESTIGUNGSELEMENTEN
PLAQUE OSSEUSE AVEC PIÈCE(S) RAPPORTÉE(S) PERMETTANT DE DIRIGER DES ATTACHES DE MANIÈRE OPTIMALE

(30) Priority: 06.02.2015 US 201562113313 P; 04.02.2016 US 201615015995; 04.02.2016 WO PCT/US2016/016647
(43) Date of publication of application: 10.08.2016
(73) Proprietor: In2Bones USA, LLC, Memphis, TN 38119 (US)
(72) Inventor: WAHL, Rebecca Hawkins, Escondido, CA 92095 (US)
(74) Representative: Dehns

(56) References cited:
- FR-A1- 3 003 155
- US-A1- 2004 068 319
- US-A1- 2010 274 293
- US-A1- 2014 222 086
- US-A1- 2014 324 108

## Description

### PRIORITY

This application claims the benefit of and priority to U.S. Provisional Application, entitled "Bone Plate With Insert(s) For Optimally Directing Fasteners," filed on February 6, 2015 having application serial number 62/113,313 (published as US 2016/0228167).

### FIELD

The field of the present disclosure generally relates to securing bones together. More particularly, the field of the present disclosure relates to an apparatus for fusing and compressing bones of the human body.

### BACKGROUND

A fusion bone plate implant may be utilized in conjunction with one or more fasteners so as to generate compression and stability at a bone interface. An implant coupled with fasteners generally serves to stabilize bones, or bone parts, relative to one another so as to promote bone fusion. In many applications, bone plates and fasteners are used to fuse bones, or bone parts, of the human body, such as bones in the foot, the ankle, the hand, the wrist, as well as various other portions of the body. Furthermore, during the course of certain medical procedures, a surgeon may immobilize one or more bones or the bone fragments by stabilizing the bones together in a configuration which approximates the natural anatomy. To this end, the surgeon may use fasteners to attach the bones to a bone plate implant so as to hold the bones in alignment with one another while they fuse together. Bone plant implants are taught in US 2004/068319, US 2014/222086 and US 2014/324108.

### SUMMARY

The present invention relates to an apparatus for fusing bones as claimed hereafter. Preferred embodiments are set forth in the dependent claims.

### BRIEF DESCRIPTION OF THE DRAWINGS

The drawings refer to embodiments of the present disclosure in which:
Figure 1A illustrates an upper perspective view of an exemplary embodiment of a bone plate with two convertible inserts and a fastener, according to the present disclosure;
Figure 1B illustrates a lower perspective view of an exemplary embodiment of a bone plate with a convertible insert and fastener in which the fastener is directed primarily in line with the longitudinal plate axis and oblique to a horizontal axis, and an additional insert that directs a fasteners primarily 90 degrees to a horizontal axis according to the present disclosure;
Figure 2A illustrates an upper perspective view of an exemplary embodiment of a bone plate with a convertible insert in which the fastener is oriented primarily 90 degrees to a horizontal axis.
Figure 2B illustrates the exemplary embodiment illustrated in Fig. 2A accompanied by a convertible insert configured for directing the fastener to a point of purchase in the bone that is approximately in line with plate long axis and oblique to horizontal axis, compressing bone parts in accordance with the present disclosure;
Figure 3A illustrates an upper perspective view of an exemplary embodiment of a bone plate with an elongated aperture for the convertible washer, according to the present disclosure;
Figure 3B illustrates a lower perspective view of the exemplary embodiment of Fig. 3A in accordance with the present disclosure;
Figure 4A illustrates an upper perspective view of an exemplary embodiment of a bone plate accompanied by a convertible insert for obliquely directing a fastener, according to the present disclosure;
Figure 4B illustrates a lower perspective view of the exemplary embodiment of Fig. 4A in accordance with the present disclosure;
Figure 5 illustrates an upper perspective view of an exemplary embodiment of a bone plate with a circular insert with an obliquely directed fastener, according to the present disclosure;
Figure 6 illustrates an exploded view of a portion of the bone plate and the circular insert illustrated in Fig. 5, in accordance with the present disclosure;
Figure 7 illustrates top views of three different circular inserts of Fig. 5 that when secured to the same implant, direct fasteners in three different orientations rotated relative to the bone plate, according to the present disclosure;
Figure 8 is an exploded view illustrating an exemplary embodiment of an insert driver configured to attach the circular insert to the bone plate illustrated in Fig. 5 in accordance with the present disclosure;
Figure 9 illustrates an upper perspective view of an exemplary embodiment of a bone plate and a circular insert with an obliquely directed fastener, according to the present disclosure;
Figure 10 illustrates an exploded view of the exemplary embodiment of the bone plate and the circular insert illustrated in Fig. 9, according to the present disclosure;
Figure 11 illustrates a bottom view of the exemplary embodiment of Fig. 10 with the circular insert installed into the bone plate in accordance with the present disclosure;
Figure 12A illustrates an close-up exploded view of a portion of the bone plate and the circular insert illustrated in Fig. 10, according to the present disclosure;
Figure 12B illustrates an exploded view of an upper portion of the bone plate and a lower portion of the circular insert illustrated in Fig. 12A, according to the present disclosure;
Figure 13A illustrates a top view of an exemplary embodiment of a bone plate and a circular insert in an exemplary retaining position which directs a fastener in a rotated orientation relative to the bone plate in accordance with the present disclosure;
Figure 13B illustrates a top view of an exemplary embodiment of a bone plate and a circular insert in an exemplary retaining position which directs a fastener in a rotated orientation relative to the bone plate, according to the present disclosure; and
Figure 13C illustrates a top view of an exemplary embodiment of a bone plate and a circular insert in an exemplary position which facilitates insertion and removal of the circular insert from the bone plate in accordance with the present disclosure.

While the present disclosure is subject to various modifications and alternative forms, specific embodiments thereof have been shown by way of example in the drawings and will herein be described in detail. The invention should be understood to not be limited to the particular forms disclosed, but on the contrary, the intention is to cover all modifications, equivalents, and alternatives falling within the scope of the present disclosure.

### DETAILED DESCRIPTION

In the following description, numerous specific details are set forth in order to provide a thorough understanding of the present disclosure. It will be apparent, however, to one of ordinary skill in the art that the invention disclosed herein may be practiced without these specific details. In other instances, specific numeric references such as "first plate," may be made. However, the specific numeric reference should not be interpreted as a literal sequential order but rather interpreted that the "first plate" is different than a "second plate." Thus, the specific details set forth are merely exemplary. The specific details may be varied from and still be contemplated to be within the scope of the present invention as defined by the claims. The term "coupled" is defined as meaning connected either directly to the component or indirectly to the component through another component. Further, as used herein, the terms "about," "approximately," or "substantially" for any numerical values or ranges indicate a suitable dimensional tolerance that allows the part or collection of components to function for its intended purpose as described herein.

In general, the present disclosure describes an apparatus for fusing bones of the human body. The apparatus comprises a bone plate which comprises a member having one or more fixation apertures and one or more insert apertures. The bone plate is comprised of a semi-rigid material, such as a biocompatible metal or Polyetheretherketone (PEEK), possessing a tensile strength suitable for immobilizing bones. An insert is configured to be retained within the insert aperture. The insert is configured to direct a fastener towards an optimal point of bone purchase for fixating or compressing the bones. One insert is configured to direct a fastener at a substantially right angle relative to the bone plate. Active compression between bones can be accomplished by driving the fastener through an elongated slot in the insert ramped on one side. It will be appreciated that the fastener shall have a smooth (non-threaded) head so that this movement within the insert slot may be achieved.

Another insert is configured to direct a fastener at a substantially oblique angle to the bone plate. Fasteners are configured to be received into the one or more fixation apertures and driven into the bones so as to immobilize the bones. In some embodiments, the plate insert aperture is configured to receive a circular insert comprising threads configured to engage with threads of the insert aperture. The threads facilitate directing the insert fastener relative to a longitudinal axis of the fusion plate, in some instances directing the insert fastener at an angle across a fusion site between the adjacent bones and in some cases directing the fastener toward an optimal point of bone purchase. In some embodiments, the circular insert comprises at least one gripping hole configured to receive a distal protrusion of an insert driver. Engaging the distal protrusion into the gripping hole and twisting the insert driver facilitates rotating the circular insert within the insert aperture so as to attach the insert securely to the plate.

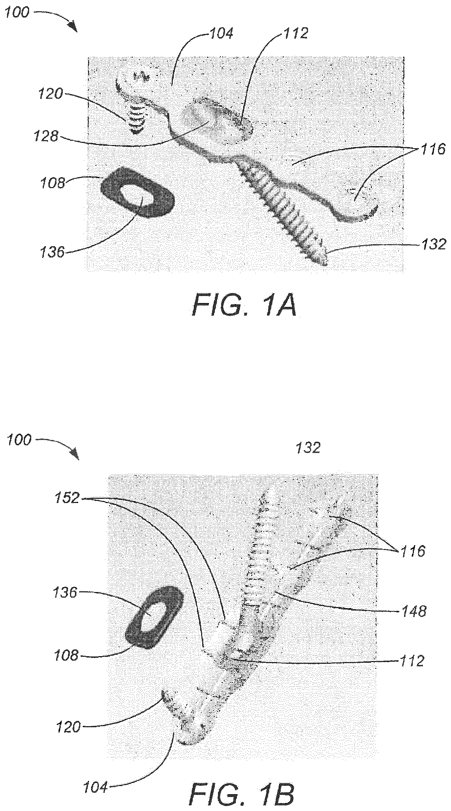

Figures 1A-1B illustrate an exemplary embodiment 100 of a bone plate 104 with a 90 degree fastener fixation insert 108 and an oblique fastener insert 112, according to the present disclosure. The bone plate 104 comprises a generally elongate member having fixation apertures 116 suitable for receiving fasteners 120. The bone plate 104 preferably is comprised of a semi-rigid material, such as a biocompatible metal or PEEK, possessing a tensile strength suitable for immobilizing adjacent bone parts of the human body.

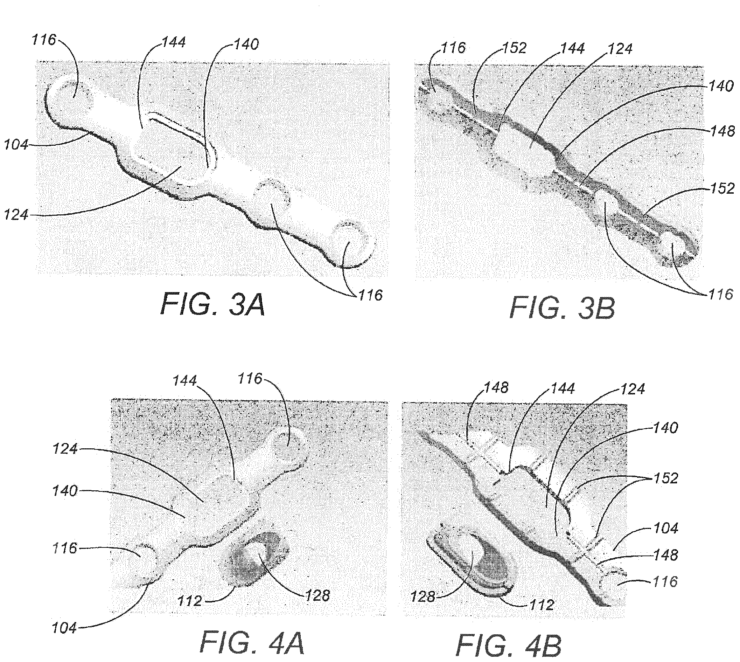

As best illustrated in Figs. 3A-4B, the bone plate 104 comprises an insert aperture 124 which is configured to receive either type of fixation insert 108,112. In the embodiment illustrated in Figs. 1A-2B, the inserts 108 and 112 are configured to be pressed into the insert aperture 124. In some embodiments, however, the inserts 108 and 112 may be configured to be mechanically retained within the insert aperture 124, such as by way of non-limiting example, through an incorporation of threads, recesses, tabs, notches, clips, as well as various protruding formations, and other similar constructs.

As illustrated in Figs. 1A-1B, the oblique fastener insert 112 comprises a convertible insert aperture 128 which is configured to receive a fastener 132, such that the threaded fastener 132 is directed at an oblique angle relative to a plane of the bone plate 104. The oblique angle of the fastener 132 facilitates compressing adjacent bones together so as to encourage bone fusion. The fastener 132 may be any component of hardware having a head configured to abut the surface of bone plate 104 and a shaft configured to secure bones together in a fixed configuration. In some embodiments, the fastener 132 may comprise a nut and bolt assembly, a pin assembly, a bone screw, or other similar fastener suitable for use in bone. In some embodiments, the fastener 132 may comprise a lag screw which includes a head that is rounded or tapered coupled to a shaft having an unthreaded portion adjacent to the head and a threaded portion that ends at a tip.

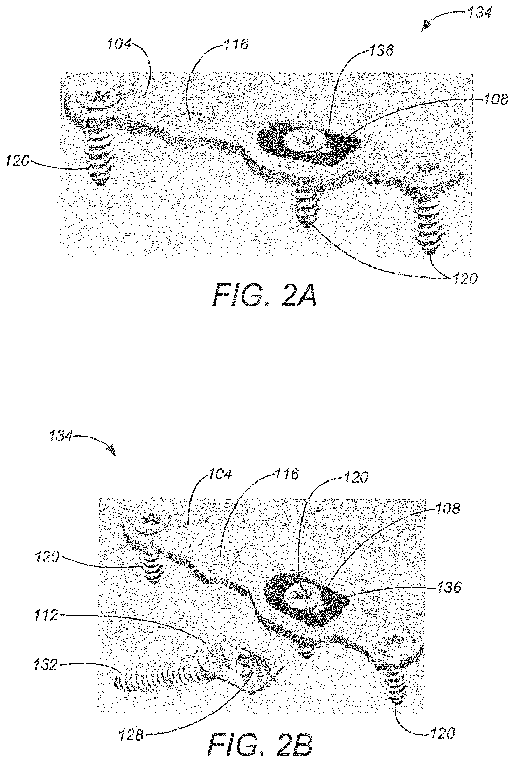

Figures 1A-1B show the bone plate 104 with the insert 112 installed into the insert aperture 124, such that the fastener 132 is directed at the oblique angle relative to the bone plate 104, as described above. Alternatively, Figs. 2A-2B show an exemplary embodiment 134 of the bone plate 104 with the fastener fixation insert 108 installed into the insert aperture 124. The insert 108 comprises a fixation aperture 136 configured to direct a fixation screw 120 at a substantially right angle relative to the bone plate 104, as best shown in Fig. 2A. It will be recognized that this insert 108 facilitates immobilizing adjacent bone parts. In some embodiments, the bone plate 104 and the insert 108 may be used with two or more fasteners 120 to fixate bones across a joint. It will be also appreciated that the fastener will have a smooth (non-threaded) so that lagging or compressing of bone fragments may be achieved.

In the embodiment illustrated in Figs. 3A-4B, the insert aperture 124 comprises a countersunk edge which includes a round portion 140 and a flat portion 144 configured to orient and receive the inserts 108, 112. It will be appreciated that the round portion 140 and the flat portion 144 are configured to orient the inserts 108, 112 advantageously within the bone plate 104. In particular, the rounded portion 140 and the flat portion 144 orient the oblique fastener insert 112 such that the fastener 132 is directed along a majority of the length of the bone plate 104, thereby providing greater support to the fasteners 132 and the bones to be fixated. Further, each of the fixation apertures 116 comprises a countersunk surface configured to receive a head of the fixation screw 120, such that the fasteners 120 fixate the bone plate 104 to the bones.

The bone plate 104 illustrated in Figs. 3A-4B, comprises a longitudinal rib 148 and a multiplicity of lateral ribs 152. The ribs 148 and 152 serve to relieve stress on the bone plate 104 arising due to contact between the plate and the bones. The longitudinal rib 148 provides structural strength to the bone plate 104 so as to substantially inhibit bending along the length of the plate. The lateral ribs 152 support the contact at the longitudinal rib 148 and reduce contact between the plate and the bone.

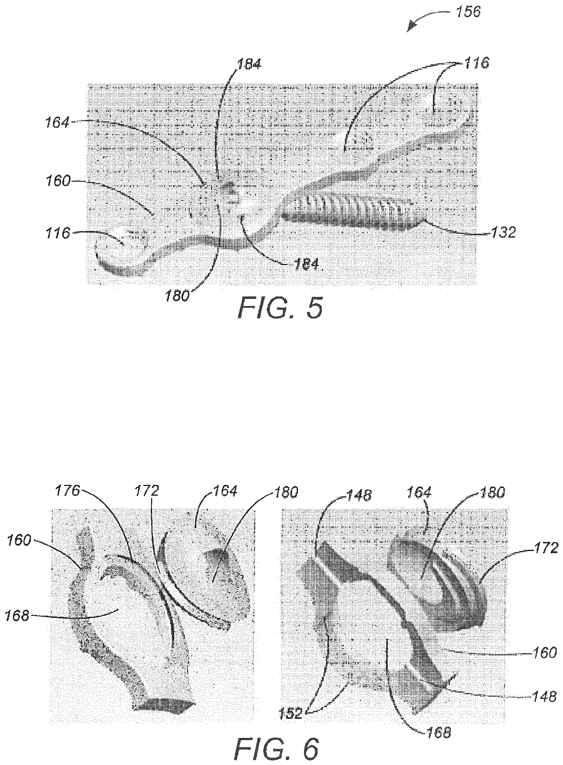

Figures 5-7 illustrate an exemplary embodiment 156 of a bone plate 160 coupled with a circular-shaped insert 164. The bone plate 160 is substantially similar to the bone plate 104, with the exception that the bone plate 160 comprises an insert aperture 168 which is configured to receive the circular insert 164. As best illustrated in Fig. 6, the circular insert 164 comprises threads 172 configured to rotatably engage with threads 176 of the insert aperture 168. As will be appreciated, the threads 172, 176 facilitate screwing the circular insert 164 into the insert aperture 168 and are configured to orient the circular insert relative to the bone plate for a desired fastener direction. In some embodiments, however, the circular insert 164 may be configured to be retained within the insert aperture 168, such as by way of the example, through an incorporation of recesses, tabs, notches, clips, various protruding formations, as well as various countersunk surfaces suitable for press-fitting the circular insert 164 into the insert aperture 168.

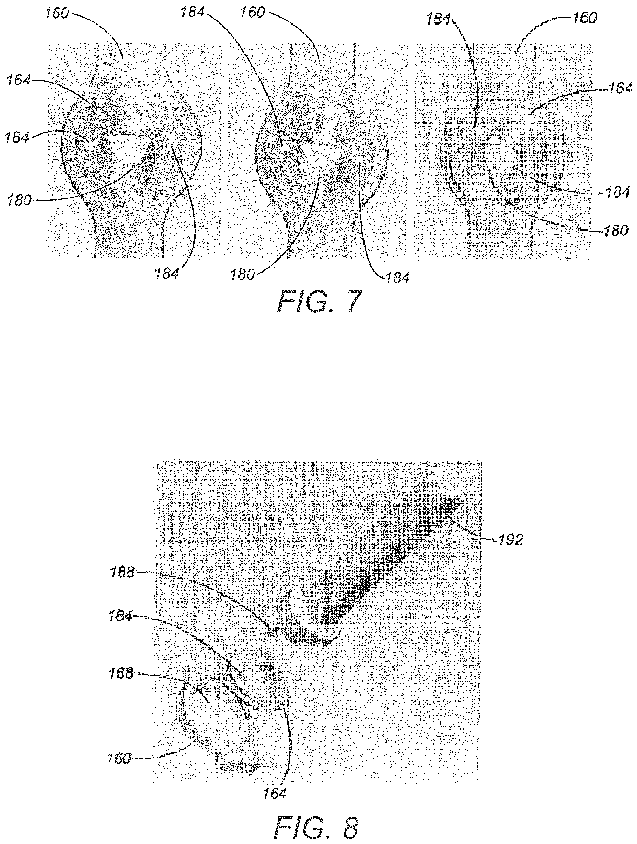

The circular insert 164 comprises an aperture 180 which is configured to receive the fastener 132, such that the fastener 132 is directed at an oblique angle relative to the plane of the bone plate 160. As described with reference to Figs. 1A-2B, the oblique angle of the compression screw 132 facilitates compressing adjacent bones together so as to encourage bone fusion. Further, the circular insert 162, as well as the threads 172, 176, facilitates rotating the compression screw 132 relative to a longitudinal dimension of the bone plate 160, as illustrated in Fig. 7. In the embodiment illustrated in Fig. 7, the insert external threads 132 may be timed to orient the insert the fastener so that it is directed at an angle ranging between substantially 0 degrees and 360 degrees relative to the longitudinal dimension of the bone plate 160. It will be appreciated that the circular insert 162 and the threads 172, 176 facilitate directing the fastener 132 at various angles across a fusion site between adjacent bones. The fastener will have a smooth (non-threaded) so that a lagging or compressing of bone fragments may be achieved.

As best shown in Figs. 5 and 7, the circular insert 164 comprises gripping holes 184. In the embodiment of Figs. 5 and 7, the circular insert 164 comprises two gripping holes 184, although any number of gripping holes may be incorporated into the circular insert without deviating from the scope of the present disclosure. As best shown in Fig. 8, the gripping holes 184 are configured to receive one or more distal protrusions 188 of an insert driver 192. It should be understood that the insert driver 192 functions as a wrench, whereby the distal protrusions 188 may be engaged into the gripping holes 184 and then the insert driver 192 may be twisted so as to rotate the circular insert 164 within the insert aperture 168. It should be further understood, therefore, that the gripping holes 184 and the insert driver 192 facilitate directing the compression screw 132 at various angles across a fusion site between adjacent bones, as discussed with reference to Fig. 7.

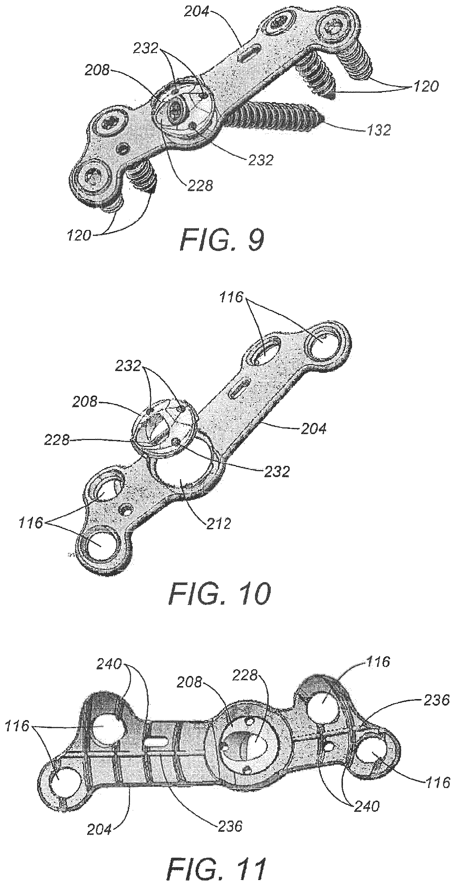

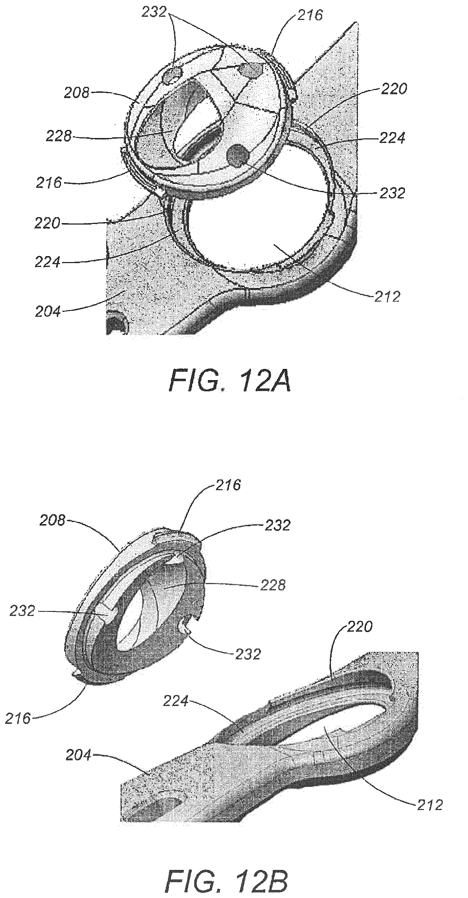

Figures 9-13C illustrate an exemplary embodiment of a bone plate 204 coupled with a circular-shaped insert 208. The bone plate 204 is substantially similar to the bone plate 160, with the exception that the bone plate 204 comprises an insert aperture 212 which is configured to receive the circular insert 208. As best illustrated in Figs. 12A and 12B, the circular insert 208 comprises circumferential protrusions 216 configured to rotatably engage with a groove 220 of the insert aperture 212. As will be appreciated, the circumferential protrusions 216 and the groove 220 facilitate engaging the circular insert 208 into the insert aperture 212 and are configured such that the circular insert may be positioned in various orientations relative to the bone plate. A countersink 224 within the insert aperture 212 maintains the circular insert 208 fastened within the bone plate 204. In some embodiments, however, the circular insert 208 may be configured to be retained within the insert aperture 212, such as by way of the example, through an incorporation of recesses, tabs, notches, clips, various protruding formations, as well as various countersunk surfaces suitable for press-fitting the circular insert 208 into the insert aperture 212, as described herein.

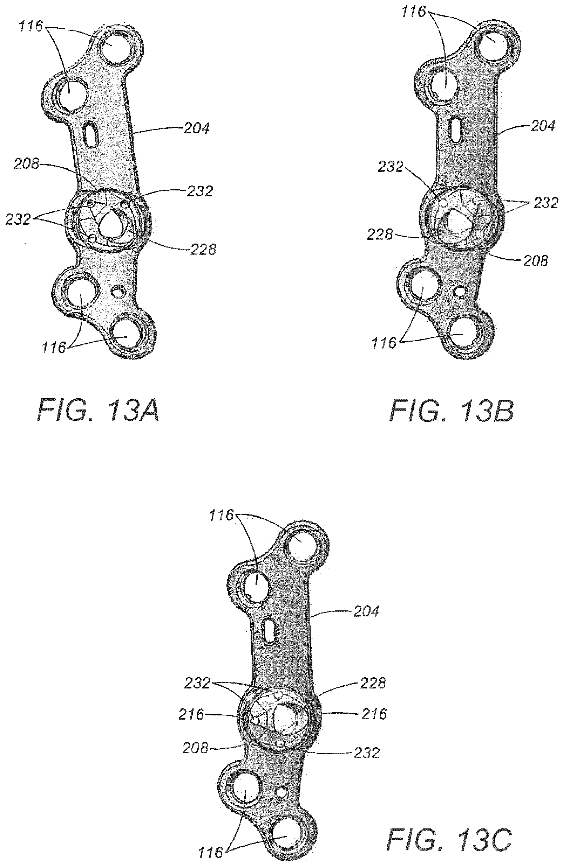

The circular insert 208 comprises an aperture 228 which is configured to receive the fastener 132, such that the fastener 132 is directed at an oblique angle relative to the plane of the bone plate 204. As described with reference to Figs. 1A-2B, the oblique angle of the fastener 132 facilitates compressing adjacent bones together so as to encourage bone fusion. Further, the circular insert 208, as well as the circumferential protrusions 216 and the groove 220, facilitates directing the fastener 132 at an angle relative to a longitudinal dimension of the bone plate 204, as illustrated in Figs. 13A and 13B. In the embodiment illustrated in Figs. 9-13B, the circumferential protrusions 216 and the groove 220 are configured to orient the circular insert 208, and thus the fastener 132 at an angle ranging between substantially 0 degrees and 360 degrees relative to the longitudinal dimension of the bone plate 204. It will be appreciated that the circular insert 208, the circumferential protrusions 216, and the groove 220 facilitate directing the fastener 132 at various angles across a fusion site between adjacent bones. Moreover, the fastener 132 comprises a smooth, non-threaded portion, such that a lagging or compressing of bone fragments may advantageously be achieved.

As best shown in Figs. 12A and 12B, the circular insert 208 comprises gripping holes 232. In the embodiment of Figs. 9-13B, the circular insert 208 comprises three gripping holes 232, although any number of gripping holes may be incorporated into the circular insert without deviating from the scope of the present disclosure. It should be appreciated that the gripping holes 232 are substantially identical to the gripping holes 184, described above in connection with Fig. 8. As with the gripping holes 184, the gripping holes 232 are configured to receive one or more distal protrusions of a suitable wrench, such as the insert driver 192. The distal protrusions 188 may be engaged into the gripping holes 232 and then the insert driver 192 may be twisted so as to rotate the circular insert 208 within the insert aperture 212. It should be further understood, therefore, that the gripping holes 232 and the insert driver 192, or other suitable wrench, facilitate directing the fastener 132 at various angles across a fusion site between adjacent bones, as discussed herein.

As best illustrated in Fig. 11, the bone plate 204 comprises a longitudinal rib 236 and a multiplicity of radial ribs 240. The ribs 236 and 240 serve to relieve stress on the bone plate 204 arising due to contact between the plate and the bones. The longitudinal rib 236 provides structural strength to the bone plate 204 so as to substantially inhibit bending along the length of the plate. The radial ribs 240 support the contact at the longitudinal rib 236 and reduce contact between the plate and the bone. It will be appreciated that the longitudinal rib 236 is substantially similar to the longitudinal rib 236, extending along the longitudinal dimension of the bone plate 104. Further, the radial ribs 240 are substantially similar to the lateral ribs 152, with the exception that each of the radial ribs 240 comprises a circular segment having a radius of curvature. In some embodiments, the radial ribs 240 all have the same radius of curvature. In some embodiments, the radial ribs 240 each has a unique radius of curvature. In some embodiments, each of the radial ribs 240 has a unique radius of curvature extending from substantially the center of the circular insert 208, along the longitudinal rib 236, to the radial rib.

Moreover, it should be recognized that the bone plate 204 is not to be limited to flat plates, but rather the bone plate 204 may comprise curvature along the longitudinal dimension of the bone plate 204, curvature along a lateral dimension of the bone plate 204, as well as a combination of curvatures along the longitudinal and lateral dimensions of the bone plate 204. In some embodiments, the curvature may change along the longitudinal and lateral dimensions of the bone plate as a function of distance from the center of the insert aperture 212. In some embodiments, the curvatures along the longitudinal and lateral dimensions of the bone plate 204 are selected to match a specific surface topology of a bone to be treated. Accordingly, it should be understood that the bone plate 204 may be implemented with any combination of topological features that fall within the scope of the claims.

It is envisioned that the embodiments discussed herein may be coupled with various surgical instruments that are configured for implanting the bone plates, inserts, and fasteners into patients. In some embodiments, the surgical instruments may include without limitation, plate trials, wires, drills, drill guides, depth gages, cup and cone reamers, screw drivers, plate benders, and the like. It is further envisioned that the bone plates, inserts, accompanying fasteners, and the selected surgical instruments are to be suitably sterilized for surgeries and packaged into sterilized containers. In some embodiments, the insert and the bone plate may be packaged in an assembled state into a first sterile container, the fasteners may be packaged into a second sterile container, and the instruments may be packaged into a third sterile container. The first, second, and third sterile containers may then be bundled together into a single, exterior container, thereby forming a convenient surgery-specific bone fusion implant package. In some embodiments, however, the bone plate and the insert may be packaged into separate sterile containers, thereby allowing a surgeon to assemble the bone plate and the insert before or during surgery. It is envisioned that other packaging techniques will be apparent to those skilled in the art without deviating from the scope of the claims.

While the invention has been described in terms of particular variations and illustrative figures, those of ordinary skill in the art will recognize that the invention is not limited to the variations or figures described. To the extent there are variations of the invention, which are within the scope of the invention defined in the claims, it is the intent that this patent will cover those variations as well. Therefore, the present disclosure is to be understood as not limited by the specific embodiments described herein, but only by scope of the appended claims.

## Claims

1. An apparatus for fusing bones of the human body, comprising:
a bone plate (104) having one or more fixation apertures (116) and an insert aperture (124);
a fastener fixation insert (108) having an aperture with a tapered, counter-bored, or countersunk internal shape configured to direct a fastener at a right angle relative to the bone plate and an oblique fastener insert (112) comprising an aperture with a tapered, counter-bored, or countersunk internal shape configured to receive a fastener, such that the fastener is directed at an oblique angle relative to a plane of the bone plate; and
the insert aperture (124) comprising a countersink edge and having a perimeter shape comprising a round portion (140) and a flat portion (144); and
one or more fixation screws (120) configured to be received into the one or more fixation apertures and driven into the bones so as to fixate or compress the bones, wherein one fixation aperture is configured to direct at least one fixation screw at a right angle relative to the bone plate; and
**characterized in that**:
the bone plate comprises a longitudinal rib (148) providing structural strength to the bone plate, and a multiplicity of lateral ribs (152) or a multiplicity of radial ribs (240) configured to minimize contact between the implant and the bone.

2. The apparatus of claim 1, wherein the bone plate is comprised of a semi-rigid material, such as a biocompatible metal or Polyetheretherketone (PEEK), possessing a tensile strength suitable for immobilizing bones.

3. The apparatus of claim 1, wherein the fastener fixation insert and the oblique fastener insert are configured to be press-fit into the insert aperture, or wherein the fastener fixation insert and the oblique fastener insert are configured to be mechanically retained within the insert aperture.

4. The apparatus of claim 1, 2 or 3 wherein aperture of each of the fastener fixation insert and the oblique fastener insert is configured to receive a smooth headed fastener.

5. The apparatus of any preceding claim, wherein the insert aperture is configured to receive a circular insert comprising threads configured to engage with threads of the insert aperture.

6. The apparatus of claim 5, wherein the circular insert comprises an aperture configured to receive a smooth-headed screw, such that the fastener is directed at an oblique angle relative to a plane of the fusion plate.

7. The apparatus of claim 6, wherein the circular insert comprises at least one engagement hole or peripheral slots configured to receive a distal protrusion of an insert driver, whereby the distal protrusion may be engaged into the engagement feature and the insert driver may be twisted so as to securely attach the circular insert within the insert aperture so as to direct the compression screw at the angle across a fusion site between the bones.

## Patentansprüche

1. Einrichtung zum Zusammenschließen von Knochen des menschlichen Körpers, umfassend:
eine Knochenplatte (104), die eine oder mehrere Fixieröffnungen (116) und eine Einsatzöffnung (124) aufweist;
einen Befestigungselement-Fixiereinsatz (108), der eine Öffnung mit einer konischen, ausgebohrten oder angesenkten Innenform aufweist, die so konfiguriert ist, dass ein Befestigungselement in einem rechten Winkel relativ zu der Knochenplatte geführt wird, und einen schrägen Befestigungselement-Einsatz (112), der eine Öffnung mit einer konischen, ausgebohrten oder angesenkten Innenform umfasst, die so konfiguriert ist, dass ein Befestigungselement so aufgenommen wird, dass das Befestigungselement in einem schrägen Winkel relativ zu einer Ebene der Knochenplatte geführt wird; und
wobei die Einsatzöffnung (124) eine Senkkante umfasst und eine Umfangsform aufweist, die einen runden Abschnitt (140) und einen flachen Abschnitt (144) umfasst; und
eine oder mehrere Fixierschrauben (120), die so konfiguriert sind, dass sie in die eine oder mehreren Fixieröffnungen aufgenommen und in die Knochen getrieben werden können, um die Knochen zu fixieren oder zu komprimieren, wobei eine Fixieröffnung so konfiguriert ist, dass sie mindestens eine Fixierschraube in einem rechten Winkel relativ zu der Knochenplatte führt; und
**dadurch gekennzeichnet, dass**:
die Knochenplatte eine Längsrippe (148), die der Knochenplatte strukturelle Festigkeit verleiht, und eine Mehrzahl von Querrippen (152) oder eine Mehrzahl von Radialrippen (240) umfasst, die so konfiguriert sind, dass sie einen Kontakt zwischen dem Implantat und dem Knochen minimieren.

2. Einrichtung nach Anspruch 1, wobei die Knochenplatte aus einem halbstarren Material besteht, wie einem biokompatiblen Metall oder Polyetheretherketon (PEEK), das eine Zugfestigkeit besitzt, die dazu geeignet ist, Knochen zu immobilisieren.

3. Einrichtung nach Anspruch 1, wobei der Befestigungselement-Fixiereinsatz und der schräge Befestigungselement-Einsatz so konfiguriert sind, dass sie in die Einsatzöffnung eingepresst werden, oder wobei der Befestigungselement-Fixiereinsatz und der schräge Befestigungselement-Einsatz so konfiguriert sind, dass sie mechanisch in der Einsatzöffnung festgehalten werden.

4. Einrichtung nach Anspruch 1, 2 oder 3, wobei eine Öffnung sowohl des Befestigungselement-Einsatzes als auch des schrägen Befestigungselement-Einsatzes so konfiguriert ist, dass sie ein Befestigungselement mit glattem Kopf aufnehmen kann.

5. Einrichtung nach einem vorstehenden Anspruch, wobei die Einsatzöffnung so konfiguriert ist, dass sie einen kreisförmigen Einsatz aufnimmt, der Gewinde umfasst, die so konfiguriert sind, dass sie in die Gewinde der Einsatzöffnung eingreifen.

6. Einrichtung nach Anspruch 5, wobei der kreisförmige Einsatz eine Öffnung aufweist, die so konfiguriert ist, dass sie eine Schraube mit glattem Kopf aufnimmt, so dass das Befestigungselement in einem schrägen Winkel relativ zu einer Ebene der Zusammenschlussplatte geführt wird.

7. Einrichtung nach Anspruch 6, wobei der kreisförmige Einsatz mindestens ein Eingriffsloch oder periphere Schlitze umfasst, das/die konfiguriert ist/sind, einen distalen Vorsprung eines Einsatztreibers aufzunehmen, wobei der distale Vorsprung in das Eingriffsmerkmal eingreifen kann und der Einsatztreiber gedreht werden kann, um den kreisförmigen Einsatz sicher in der Einsatzöffnung zu befestigen, um die Kompressionsschraube in dem Winkel über eine Zusammenschlussstelle zwischen den Knochen zu führen.

## Revendications

1. Appareil pour fusionner les os du corps humain, comprenant :
une plaque osseuse (104) présentant une ou plusieurs ouvertures de fixation (116) et une ouverture d'insert (124) ;
un insert de fixation d'attache (108) présentant une ouverture avec une forme interne conique, lamée ou fraisée configurée pour diriger une attache à angle droit par rapport à la plaque osseuse et un insert d'attache oblique (112) comprenant une ouverture avec une forme interne conique, lamée ou fraisée configurée pour recevoir une attache, de telle sorte que l'attache soit dirigée selon un angle oblique par rapport à un plan de la plaque osseuse ; et
l'ouverture d'insert (124) comprenant un bord fraisé et présentant une forme de périmètre comprenant une partie ronde (140) et une partie plate (144) ; et
une ou plusieurs vis de fixation (120) configurées pour être reçues dans les une ou plusieurs ouvertures de fixation et enfoncées dans les os de manière à fixer ou comprimer les os, dans lequel une ouverture de fixation est configurée pour diriger au moins une vis de fixation à angle droit par rapport à la plaque osseuse ; et
**caractérisé en ce que** :
la plaque osseuse comprend une nervure longitudinale (148) fournissant une résistance structurelle à la plaque osseuse, et une multiplicité de nervures latérales (152) ou une multiplicité de nervures radiales (240) configurées pour réduire au minimum le contact entre l'implant et l'os.

2. Appareil selon la revendication 1, dans lequel la plaque osseuse est constituée d'un matériau semi-rigide, tel qu'un métal biocompatible ou du polyétheréthercétone (PEEK), possédant une résistance à la traction adaptée à l'immobilisation des os.

3. Appareil selon la revendication 1, dans lequel l'insert de fixation d'attache et l'insert d'attache oblique sont configurés pour être ajustés par pression dans l'ouverture d'insert, ou dans lequel l'insert de fixation d'attache et l'insert d'attache oblique sont configurés pour être retenus mécaniquement dans l'ouverture d'insert.

4. Appareil selon la revendication 1, 2 ou 3, dans lequel l'ouverture de chacun de l'insert de fixation d'attache et de l'insert d'attache oblique est configurée pour recevoir une attache à tête lisse.

5. Appareil selon une quelconque revendication précédente, dans lequel l'ouverture d'insert est configurée pour recevoir un insert circulaire comprenant des filetages configurés pour venir en prise avec des filetages de l'ouverture d'insert.

6. Appareil selon la revendication 5, dans lequel l'insert circulaire comprend une ouverture configurée pour recevoir une vis à tête lisse, de telle sorte que l'attache soit dirigée selon un angle oblique par rapport à un plan de la plaque de fusion.

7. Appareil selon la revendication 6, dans lequel l'insert circulaire comprend au moins un trou de mise en prise ou des fentes périphériques configuré(es) pour recevoir une saillie distale d'un dispositif de mise en place d'insert, selon lequel la saillie distale peut être mise en prise dans l'élément de mise en prise et le dispositif de mise en place d'insert peut être tordu de manière à fixer solidement l'insert circulaire à l'intérieur de l'ouverture d'insert de manière à diriger la vis de compression selon l'angle à travers un site de fusion entre les os.
